Europäisches Patentamt

European Patent Office

Office européen des brevets

(11) Publication number: **0 145 092**
**B1**

(12) EUROPEAN PATENT SPECIFICATION

(45) Date of publication of patent specification: **30.09.87**

(21) Application number: **84201779.0**

(22) Date of filing: **30.11.84**

(51) Int. Cl.⁴: **C 12 N 9/38, A 23 C 9/12**

(54) Process for the preparation of a germ-free solution of a lactose-splitting enzyme and its use.

(30) Priority: **02.12.83 NL 8304153**

(43) Date of publication of application:
**19.06.85 Bulletin 85/25**

(45) Publication of the grant of the patent:
**30.09.87 Bulletin 87/40**

(84) Designated Contracting States:
**AT BE CH DE FR GB IT LI LU NL SE**

(56) References cited:
**GB-A-1 477 087**

**VOEDINGSMIDDELEN TECHNOLOGIE, vol. 13, no. 5, March 1970, page 23, Zeist, NL; "Melk met gereduceerd lactose gehalte"**

**CHEMICAL ABSTRACTS, vol. 99, no. 19, November 1983, page 506, no. 157093z, Columbus, Ohio, US**

**FOOD SCIENCE & TECHNOLOGY ABSTRACTS, abstract no. 79051743, Gist-Brocades NV, Delft, NL; H.H. NIJPELS: "Maxilact: milk for millions. Why milk with a low lactose content ?"**

(73) Proprietor: **Gist - Brocades N.V.**
**Wateringseweg 1 P.O. Box 1**
**NL-2600 MA Delft (NL)**

(72) Inventor: **Bijl, Hendrik Louis**
**Insulindestraat 72**
**NL-3131 ZW Vlaardingen (NL)**
Inventor: **Repelius, Cornelis**
**Vliervinder 5**
**NL-2317 JR Leiden (NL)**

(74) Representative: **Huygens, Arthur Victor, Dr. et al**
**c/o Gist-Brocades N.V. Patent & Trademarks Department Martinus Nijhofflaan 2 PO Box 1 NL-2600 MA Delft (NL)**

(56) References cited:
**FOOD SCIENCE & TECHNOLOGY ABSTRACTS, abstract no. 78003500, Dep. of Food Sci., Chem. Cent., Univ. of Lund, Lund, SE; A. DAHLQVIST et al.: "Hydrolysis of lactose in milk and whey with minute amounts of lactase"**

# 0 145 092

## Description

This invention relates to a process for the preparation of a germ-free solution of a lactose-splitting enzyme, and to its use in preparing milk and milk products with a reduced lactose content.

Lactose (milk sugar) is a disaccharide present in dairy products and more particularly in milk, skimmed milk, cream and other milk products. These products contain lactose in an amount of approximately 4—5%. The non-absorbable lactose is usually broken down in the body into the absorbable monosaccharides glucose and galactose. This break-down is generally brought about by a natural enzyme, named β-galactosidase or lactase, present in the intestinal wall in the human body (and in other mammals).

In large areas of the world humans and animals suffer from so-called lactose-intolerance, which is caused by the total or partial absence from their digestive system of lactase for the break-down of lactose-containing products. Therefore the lactose is either not broken down, or is insufficiently broken down into glucose and galactose which can be absorbed: the non-absorbable lactose then causes intestinal trouble.

The phenomenon of lactose intolerance has been recognized for many years. Because of its amino acid constitution milk protein has a large food value. Attempts have been made to prepare lactose-poor or lactose-free milk and milk products for sufferers from lactose intolerance by adding lactase to milk and milk products. An extra advantage of the addition of lactase may be that the products treated in this way are sweeter.

It is possible to add the enzyme and to allow it to react before preserving milk and milk products by pasteurization or sterilization. However, during the conservation the well-known Maillard-reaction takes place and spoils the taste of the milk and milk products.

In British Patent Specification 1,477,087 a process is described for treating milk and milk products with a lactose-splitting enzyme, adding the enzyme to the previously pasteurized or sterilized milk just before packing, whereafter the packing is closed. The desired reaction of the enzyme with the lactose present then takes place in the closed packing. As the enzyme is added after pasteurization or sterilization, the Maillard-reaction does not occur under those circumstances. It will be clear that the enzyme itself has to be made germ-free before adding it to the sterilized milk and that the addition to the milk and/or milk products must be carried out under aseptic conditions and with sterile apparatus.

In published Swedish Patent Application 7800137 a process is described for the sterilization of lactase by irradiation or chemical treatment, for instance with ethylene oxide. According to the description the enzyme may be sterilized in dissolved form in closed aseptic packages as well as in solid form. In the latter case the sterilized lactase is then dissolved in a previously sterilized solvent. The container with the sterilized lactase solution is connected to a dosing device through which the sterile enzyme solution is added to the sterilized milk, which is then packed. However, it appeared that both sterilization methods involve large activity losses of the lactase enzyme and a long period of time is required for the sterilization.

In the journal Voedingsmiddelentechnologie *13* (1980), 23 the method described in British Patent Specification 1,477,087 is further illustrated. The germ-poor lactase, usually supplied to the dairy processing industry in an aqueous solution to which one or more stabilizing agents, e.g. glycerol, are added, is filtered before use. The filtered enzyme solution is pumped through a sterile filter, injected via a dosing device into a production line of previously sterilized milk and then mixed with the milk. Finally the mixture is divided and packed under aseptic conditions in uniform packs.

However, in practice in the system just described the sterile filter often blocks. The blockages are mostly due to degradation of proteins and polysaccharides (gums), which may remain in the enzyme solution in spite of the purification. The degradation phenomena generally increase the longer the enzyme is kept prior to use and may be promoted because there may be a considerable period of time between the production of the enzyme by the enzyme manufacturer and its use by the dairy processing industry. Protease present in the lactase may also play a role in the degradation processes.

It will be clear that repeatedly replacing or cleaning of sterile filters seriously impedes the efficiency of the process. Not only does the process have to be stopped in order to remove the sterile filter, but also the whole system has to be sterilized anew before it can be put into use again.

For many years attempts have been made to solve the germ filtration problem in several ways, but these efforts either did not lead to the desired results or were too expensive for an economical process on an industrial scale. Cascade filtration was tried, i.e. filtration with the aid of a series of membrane filters with decreasing pore size, but surprisingly these filters also proved to clog after a relatively short time. It was also proposed to centrifuge the enzyme solution before passage through the sterile filter, but this method equally did not give optimal results and also made the process considerably more expensive. Use of other recovery methods, for instance to achieve a further clarification of the enzyme solution also did not solve the problem of blocked filters.

It has now been found that the above-described filtration problem can be overcome by making the enzyme germ-free shortly after the fermentation and the usual recovery and purification and optionally by keeping it sterile in a solution before adding it to the milk.

The invention therefore provides a process for the preparation of a germ-free solution of a lactose-splitting enzyme characterized in that a solution of the lactose-splitting enzyme is made germ-free by sterile filtration after the recovery and purification of a solution of lactose-splitting enzyme produced by fermentation, but before the formation of degradation products sufficient to clog the sterile filter.

2

# 0 145 092

The process according to the invention has the additional advantage that the sterilization of the enzyme solution can be carried out by the enzyme producer himself. Up to now the different dairy processing industries each used to subject the enzyme solution supplied to sterile filtration or possibly sterilized it in another way.

The enzyme solution can be made germ-free by any known methods which all fall within the scope of the invention. Preferably, after the usual recovery, purification, autolysis and filtration the enzyme solution is made germ-poor before it is subjected to sterile filtration. It is preferred to make the solution germ-poor with a germ reduction filter, for instance SIETZ Supra EKS® filter pads. It is preferable to make the solution germ-poor immediately after the aforementioned steps. Preferably, the solution is also subjected to ultrafiltration to concentrate the enzyme before sterile filtration.

The sterile filtration (germ filtration) is preferably carried out with one or more membrane filters. A suitable filter is for instance a membrane filter with a pore size of 0.22 µm.

The timing of the germ filtration of the enzyme solution is not critical but should be chosen so that no considerable amount of degradation products has yet been formed to cause clogging of the filter. It is therefore recommended to carry out the germ filtration shortly after the recovery and purification. Preferably, the germ filtration is carried out within 14 days and more preferably within 5 days of the recovery and purification.

Before or after the sterilization of the enzyme solution by sterile filtration one or more solvents or other additives may be added, for example to bring the enzyme activity to the desired level and to further stabilize the enzyme. Suitable solvents are, for example, sorbitol and glycerol. Suitable additives, which stabilise the enzyme are, for example, hydrolysed lactose, glucose, mannitol and salt buffers. Also, additives may be added to sweeten further the milk and milk products. Such additive is, for example, invertase, which may be added to or is already present in the enzyme solution. Preferably, the addition is carried out after the sterilization, the added compounds being of course previously sterilized. A preferred solvent, which at the same time contributes to the stabilization of the enzyme, is glycerol. The final glycerol-water ratio is not critical but is preferably between 1:9 and 4:1 by volume, and particularly between 1:3 and 1:1.

In a suitable embodiment which is illustrative of the invention an aqueous fermentation product containing lactase is subjected to autolysis and subsequently filtered, preferably with filter aids. If necessary, the filtrate is treated with active charcoal, and passed through a germ reduction filter (a so-called polish filter) to make the solution germ-poor. Ultrafiltration then takes place to concentrate the enzyme, after which glycerol is added (about 50 wt.%). The solution thus obtained is again passed through a germ reduction filter (SEITZ Supra EKS filter pads) and then in line through a sterile filter (a membrane filter with a pore size of 0.22 µm). Finally the solution is passed into a sterile container which is immediately sealed.

In another suitable embodiment about 20 wt.% of glycerol is added to the filtrate, after the autolysis, filtration, polish filtration and ultrafiltration as described above. The solution obtained is then passed through a germ reduction filter and subsequently through a sterile filter. The solution is then adjusted to the required concentration (about 1:1) with sterile glycerol and is packed under sterile conditions.

In a preferred embodiment about 50 wt.% of glycerol is added after the prefiltration and sterile filtration steps as described above. An advantage of the latter method with regard to the above-mentioned methods is that the solution before the sterile filtration is less viscous, which permits a faster filtration.

The invention further relates to a process for the preparation of milk or a milk product with a reduced lactose content (which also means a sweeter product) by adding a germ-free solution of a lactose-splitting enzyme, prepared by the process of the invention.

The sterile enzyme solution can be preserved in a sterile container before being used by the dairy processing industry. Sterile containers in which liquids may be preserved have been described frequently in the literature. Preferably a container is used which can be easily connected to the dosing pump into a milk production line, for instance as described in the previously mentioned article in the journal Voedingsmiddelentechnologie. The connection of the container to the dosage pump must, of course, be carried out under germ-free circumstances. The sterile filter between the sterile enzyme solution and the production line as described in the processes of the prior art can be omitted when a germ-free solution of enzyme obtained by a process according to the invention is employed. Precipitate which may occur because of degradation processes does not clog the dosage pump and is totally harmless in nature. Moreover the enzyme concentration in the milk is so low that the amount of precipitate in the final product is insignificant.

The following Examples illustrate the invention.

## Example 1

Sterile filtration of a solution containing lactase.

Maxilact®, a commercial aqueous fermentation product of Gist-Brocades, containing lactase was prefiltered over SEITZ K5, SEITZ EK and MILLIPORE RAWP (pore size 1.2 µm) filter pads. For the final filtration the solution was passed through a Millipore GSWP membrane filter (pore size 0.22 µm) and collected in a sterile bottle. The solution was then adjusted to the required concentration with sterile glycerol. The sterility of four bottles with different glycerol concentration (and activity) is given below.

3

0 145 092

| glycerol concentration (wt.%) | activity (%)* | germ count/ml |
| --- | --- | --- |
| 0 | 91 | <1 |
| 20 | 80 | <1 |
| 50 | 49 | <0.01 |
| 50 | 49 | <0.01 |

* related to the activity of the starting product.

A clotting test and a taste panel test did not reveal any deviation from the starting product.

Example 2

Use of sterile Maxilact® in a dairy plant.

A solution containing 50 wt.% glycerol was used in a test in a dairy plant. This solution was injected via a dosing device in a production line of previously sterilized milk and mixed in a mixer with the milk. Finally, the mixture was divided and packed under aseptic conditions in packages of 1 litre.

The sterility of the milk after 7 days storage at 30°C was 100%. More than 85% of the lactose was hydrolysed.

## Claims

1. Process for the preparation of a germ-free solution of a lactose-splitting enzyme characterized in that a solution of the lactose-splitting enzyme is made germ-free by sterile filtration after the recovery and purification of a solution of lactose-splitting enzyme produced by fermentation, but before the formation of degradation products sufficient to clog the sterile filter.

2. Process according to claim 1, in which the enzyme solution is made germ-poor immediately after the recovery and purification and before the sterile filtration

3. Process according to claim 1 or 2, in which the sterile filtration is carried out within 14 days of the recovery and purification.

4. Process according to claim 1, 2 or 3, in which one or more solvents and/or additives are added to the enzyme solution.

5. Process according to claim 4, in which glycerol is added.

6. Process according to claim 4, in which about 50 wt.% of glycerol is added to the enzyme solution after the sterile filtration.

7. Process according to any one of Claims 1 to 6 comprising the additional step of introducing the resulting germ-free solution of a lactose-splitting enzyme into a sterile container in a manner known per se.

8. Process for the preparation of milk or a milk product with reduced lactose content by the addition of a germ-free solution of a lactose-splitting enzyme, prepared by the process according to any one of claims 1—7.

## Patentansprüche

1. Verfahren zur Herstellung einer keimfreien Lösung eines Lactose spaltenden Enzyms, dadurch gekennzeichnet, dass eine Lösung des Lactose spaltenden Enzyms nach der Gewinnung und Reinigung einer Lösung von Lactose spaltendem Enzym, das durch Fermentierung erzeugt wurde, aber vor der Bildung von Abbauprodukten, die genügen, um das Sterilfilter zu verstopfen, durch Sterilfiltration keimfrei gemacht wird.

2. Verfahren nach Anspruch 1, dadurch gekennzeichnet, dass die Enzymlösung unmittelbar nach der Gewinnung und Reinigung und vor der Sterilfiltration keimfrei gemacht wird.

3. Verfahren nach Anspruch 1 oder 2, dadurch gekennzeichnet, dass die Sterilfiltration innerhalb von 14 Tagen nach der Gewinnung und Reinigung ausgeführt wird.

4. Verfahren nach Anspruch 1, 2 oder 3, dadurch gekennzeichnet, dass ein oder mehrere Lösungsmittel und/oder Additive zu der Enzymlösung zugesetzt werden.

5. Verfahren nach Anspruch 4, dadurch gekennzeichnet, dass Glycerin zugesetzt wird.

6. Verfahren nach Anspruch 4, dadurch gekennzeichnet, dass nach der Sterilfiltration ca. 50 Gew.-% Glycerin zu der Enzymlösung zugesetszt werden.

7. Verfahren nach einem der Ansprüche 1 bis 6, gekennzeichnet durch die zusätzliche Stufe, dass die resultierende keimfreie Lösung eines Lactose spaltenden Enzyms in an sich bekannter Weise in einen sterilen Behälter eingebracht wird.

8. Verfahren zur Herstellung von Milch oder einem Milchprodukt mit reduziertem Lactosegehalt durch Zugabe einer keimfreien Lösung eines Lactose spaltenden Enzyms, die nach dem Verfahren gemäss einem der Ansprüche 1 bis 7 hergestellt ist.

4

**Revendications**

1. Procédé de préparation d'une solution exempte de germes qui contient une enzyme scindant le lactose, caractérisé en ce qu'une solution de l'enzyme scindant le lactose est rendue exempte de germes par filtration stérile après l'isolement et la purification d'une solution d'enzyme scindant le lactose produite par fermentation, mais avant la formation de produits de dégradation en quantité suffisante pour colmater le filtre stérile.

2. Procédé suivant la revendication 1, dans lequel la solution d'enzyme est rendue pauvre en germes immédiatement après l'isolement et la purification et avant la filtration stérile.

3. Procédé suivant la revendication 1 ou 2, dans lequel la filtration stérile est exécutée dans les quatorze jours de l'isolement et de la purification.

4. Procédé suivant la revendication 1, 2 ou 3, dans lequel un ou plusieurs solvants et/ou additifs sont ajoutés à la solution d'enzyme.

5. Procédé suivant la revendication 4, dans lequel du glycérol est ajouté.

6. Procédé suivant la revendication 4, dans lequel environ 50% en poids de glycérol sont ajoutés à la solution d'enzyme après la filtration stérile.

7. Procédé suivant l'une quelconque des revendications 1 à 6, comprenant le stade supplémentaire d'introduire d'une manière en elle-même connue, dans un récipient stérile, la solution résultante exempte de germes qui contient une enzyme scindant le lactose.

8. Procédé de préparation de lait ou d'un produit laitier à teneur réduite en lactose, par addition d'une solution exempte de germes qui contient une enzyme scindant le lactose, préparée par le procédé suivant l'une quelconque des revendications 1 à 7.